# EUROPEAN PATENT SPECIFICATION

(11) **EP 4 560 568 B1**
(45) Date of publication and mention of the grant of the patent: **15.04.2026**
(21) Application number: 23211142.7
(22) Date of filing: 21.11.2023
(51) Int. Cl.: G06T 7/00, A61B 6/03

(54) **METHOD FOR EVALUATING THE EXPLOITABILITY OF 4D-TOMOGRAPHIC IMAGE DATA, COMPUTER PROGRAM PRODUCT AND SCANNER DEVICE**
VERFAHREN ZUR BEWERTUNG DER VERWERTBARKEIT 4D-TOMOGRAPHISCHER BILDDATEN, COMPUTERPROGRAMMPRODUKT UND SCANNERVORRICHTUNG
PROCÉDÉ D'ÉVALUATION DE L'EXPLOITABILITÉ DE DONNÉES D'IMAGE DE TOMOGRAPHIE 4D, PRODUIT DE PROGRAMME INFORMATIQUE ET DISPOSITIF DE BALAYAGE

(43) Date of publication of application: 28.05.2025
(73) Proprietor: Siemens Healthineers AG, 91301 Forchheim (DE)
(72) Inventor: Dickmann, Jannis, 91052 Erlangen (DE); Vega, Fernando, 91056 Erlangen (DE)
(74) Representative: Siemens Healthineers Patent Attorneys

(56) References cited:
- TIANCHENG HE ET AL: "Spatial- Temporal Image- Constrained Lung 4D- CT Reconstruction for Radiotherapy Planning", 1 January 2015, CLIP 2014, LNCS 8680, Switzerland, ISBN: 978-3-540-74549-5, XP047302627, DOI: 10.1007/978-3-319-13909-8_16
- YAMAMOTO T ET AL: "Retrospective Analysis of Artifacts in Four-Dimensional CT Images of 50 Abdominal and Thoracic Radiotherapy Patients", INTERNATIONAL JOURNAL OF RADIATION: ONCOLOGY BIOLOGY PHYSICS, PERGAMON PRESS, USA, vol. 72, no. 4, 15 November 2008 (2008-11-15), pages 1250 - 1258, XP025573186, ISSN: 0360-3016, [retrieved on 20080925], DOI: 10.1016/J.IJROBP.2008.06.1937
- WANG H ET AL: "Improving Soft-Tissue Contrast in Four-Dimensional Computed Tomography Images of Liver Cancer Patients Using a Deformable Image Registration Method", INTERNATIONAL JOURNAL OF RADIATION: ONCOLOGY BIOLOGY PHYSICS, PERGAMON PRESS, USA, vol. 72, no. 1, 1 September 2008 (2008-09-01), pages 201 - 209, XP023977460, ISSN: 0360-3016, [retrieved on 20080820], DOI: 10.1016/J.IJROBP.2008.04.054

## Description

The invention relates to a method for evaluating the exploitability of 4D-tomographic image data. This method is situated in the field of medical technology, specifically in the domains of medical imaging and the utilization of medical images in radiation therapy.

Time-resolved 4D computed tomography imaging (4DCT) is essential for radiotherapy treatment planning of moving tumors. It generates a 3D-tomographic image at multiple time-points throughout a patient's breathing cycle. However, technical challenges often plague this process. Irregularities in a patient's breathing pattern during 4DCT acquisition, or a mismatch between the scanner's rotation time and the patient's breath rate, can result in severe image artifacts. These artifacts, including stack transition artifacts, interpolation artifacts, and motion artifacts, have the potential to render the acquired images unsuitable for treatment planning. The consequences of these issues are far-reaching. If these artifacts go undetected during the initial 4DCT scan and are only discovered during the treatment planning phase, it necessitates a complete repetition of the scan, leading to significant increases in both cost and time, patient discomfort, and potentially a compromised treatment outcome due to the delay. Therefore, the development of an automated algorithm to detect and locate such image artifacts is of utmost importance. This algorithm can pinpoint the exact location of the artifact, providing the treating physician with the necessary information to make an informed decision about whether a scan repetition is required.

In the current landscape of clinical practice, the evaluation of 4DCT images takes place post-scan, typically conducted by therapists or physicians. During this evaluation, a critical decision must be made promptly - whether to proceed with the acquired data or, in the presence of artifacts that could significantly impact the subsequent treatment plan, initiate a rescan of the patient. Given the inherent complexity of 4DCT images, with their high-dimensional nature, a comprehensive manual review of the entire dataset is practically unfeasible. Instead, healthcare professionals resort to examining only a fraction of the image data, typically focusing on a subset of crucial slices and specific time points.

This selective review approach, while efficient in terms of time, is not without its shortcomings. The inherent risk lies in the potential oversight of image artifacts, which might remain undetected during the initial evaluation but surface later during the treatment planning phase. Such a delay in the discovery of these artifacts can lead to several drawbacks. It amplifies the necessity for a repeat scan, imposing additional financial costs and prolonged treatment timelines on patients. Furthermore, it can introduce heightened levels of patient discomfort due to repeated procedures, and potentially compromise the efficacy of the treatment itself. The limitations of this current practice emphasize the urgent need for more effective and comprehensive solutions in the assessment of 4DCT images to enhance the overall quality and efficiency of patient care.

The document US10803587B2 describes a method including: capturing a respiratory movement of the patient; determining a respiration-correlated parameter, from the respiratory movement of the patient; specifying a measurement region of the imaging examination the measurement region including at least one z-position; automatically calculating at least one measurement parameter in accordance with the respiratory movement, using the respiration-correlated parameter as an input parameter; and performing the imaging examination of the patient, in accordance with the at least one measurement parameter in the measurement region via the computer tomograph, to capture the projection data, wherein the projection data, when captured, depicts the respiratory cycle of the patient at the at least one z-position over the complete time duration of the respiratory cycle.

The document Tiancheng He et al: "Spatial Temporal Image Constrained Lung 4D- CT Reconstruction for Radiotherapy Planning" (1 January 2015 (2015-01-01), CLIP 2014, LNCS 8680, Switzerland DOI: 10.1007/978-3-319-13909-8_16) discloses a Bayesian-based method for improving 4D computed tomography (4D-CT) reconstruction in thoracic radiotherapy planning. The approach addresses artifacts caused by irregular breathing cycles by incorporating spatial continuity and temporal smoothness of anatomical structures such as the chest surface, bones, vessels, and lungs. By optimizing the assignment of axial images to respiratory phases and applying nonuniform cubic B-spline interpolation, the method achieves smoother and more anatomically consistent helical 4D-CT images. Experimental results on forty lung cancer patients demonstrated that this technique produces fewer artifacts and better structural continuity compared to existing surrogate-based, image-matching, and chest-surface-constrained methods.

The document YAMAMOTO T ET AL: "Retrospective Analysis of Artifacts in Four _Dimensional CT Images of 50 Abdominal and Thoracic Radiotherapy Patients", INTERNATIONAL JOURNAL OF RADIATION: ONCOLOGY BIOLOGY PHYSICS, PERGAMON PRESS, USA, vol. 72, no. 4, 15 November 2008 (2008-11-15), pages 1250-1258) discloses a study from Stanford University that quantifies the type, frequency, and magnitude of artifacts in 4D computed tomography (4D-CT) images acquired using a multislice cine method. In 50 patients with thoracic or abdominal cancers, 90% showed at least one artifact, mainly in the diaphragm or heart, with an average size of 11.6 mm. The presence and severity of artifacts were significantly related to variations in patients' breathing patterns, particularly abdominal displacement. The study concludes that artifact occurrence in current 4D-CT imaging is alarmingly high, indicating a strong need for improved acquisition and reconstruction methods.

The invention effectively resolves a series of critical challenges in the field of tomographic imaging. Firstly, it guarantees the comprehensive detection of artifacts within time-resolved tomography datasets, leaving no room for oversight. Moreover, this innovative solution facilitates real-time artifact detection, ensuring that potential issues are identified as they arise, rather than in hindsight. Furthermore, it achieves these objectives with exceptional efficiency, making it executable on conventional computers or via cloud-based systems, utilizing standard system resources. Crucially, the system enables artifact detection while the patient remains in the scanner, allowing for immediate data acquisition and eliminating the need for additional scanning sessions. In addition, it empowers the scanner operator to view only those datasets or 3D data that exhibit artifacts, streamlining the decision-making process regarding the necessity of further imaging. Importantly, this invention ensures that all scans are meticulously scrutinized for artifacts, leaving no room for oversight or error in the assessment process. Ultimately, it eliminates the possibility of artifacts escaping the attention of the operator, thus enhancing the overall quality and reliability of tomographic image data.

Independent of the grammatical term usage, individuals with male, female or other gender identities are included within the term.

The invention concerns a method for evaluating the exploitability of 4D-tomographic image data, as disclosed in claim 4D-tomographic image data refers preferably to a dynamic dataset generated by combining 3D-tomographic images taken at various time points. This technique is particularly prominent in medical imaging, encompassing modalities such as Computed Tomography (CT), Magnetic Resonance Imaging (MRI), Positron Emission Tomography (PET), and others. The term "4D" signifies the addition of time as the fourth dimension, allowing the visualization of anatomical structures or pathological processes over a specified period. In the context of medical imaging, 4D imaging, especially time-resolved 3D-tomography, is indispensable for understanding organ or tumor motion, which is often vital for precise diagnosis and treatment planning. The 4D-tomographic image data are preferably configured as 4D-CT image data or 4D-MR image data.

In medicine, 4D-tomographic image data plays a pivotal role in radiotherapy planning and gating. It is particularly beneficial in managing moving organs and tumors, a common challenge in oncology. By capturing the real-time movement of internal structures, clinicians can optimize radiation therapy, ensuring that the tumor receives the prescribed dose while minimizing damage to healthy tissues. This is achieved through gating, a technique that synchronizes radiation delivery with the patient's respiratory or cardiac cycle. For example, in lung cancer treatment, 4D imaging helps create treatment plans that adjust for lung tumor motion during breathing.

Artifacts in 3D and 4D-tomographic image data can take various forms, with motion-based artifacts being of particular concern. In 4D-tomographic image data just some 3D-tomographic image data can have artifacts, especially just at some time points. The artifacts include blurring, misalignment, and distortions in the images caused by patient motion during image acquisition. For instance, respiratory motion can lead to image smearing, where the structures appear elongated or distorted. The artifacts compromise the accuracy and reliability of the images. They can result in incorrect tumor localization, making it challenging to precisely plan and administer radiation therapy. As a result, tomographic image data with these artifacts cannot be used for radiotherapy or may require extensive post-processing, which can be time-consuming and often insufficient in mitigating the impact of motion artifacts.

The 4D-tomographic image data acquired by scanners like CT, MRI, and PET can be provided by the scanner and/or can be received through various means. The 4D-tomographic image data can be transferred through a physical cable connection, such as USB, Ethernet, or proprietary connectors. This method ensures a direct and reliable link between the scanner and the receiving device, this makes the method of the invention particularly fast and reliable. Alternatively, wireless data transmission can be used. Wi-Fi or Bluetooth connectivity allows for cable-free data transfer, which can be especially convenient for mobile or portable scanners. Network-based data transfer is common, where scanners are connected to local area networks (LAN) or wide area networks (WAN). This method enables remote access, storage, and retrieval of image data.

In terms of data formats, the 4D-tomographic image data are preferably provided and/or received in standard formats such as DICOM (Digital Imaging and Communications in Medicine) or FHIR. This format ensures compatibility with medical imaging systems and allows for seamless integration with Picture Archiving and Communication Systems (PACS).

The 4D-tomographic image data can be provided and/or received as raw data or as preprocessed data. Raw data can be configured as raw projection data. Raw data requires further processing using software to generate tomographic images. While this approach offers flexibility, it necessitates significant post-processing. Preprocessed data preferably comprise reconstructed 2D or 3D images. These images have already undergone initial processing, including filtering and reconstruction. While more user-friendly and suitable for clinical use, they may be less flexible for certain research applications.

The step receiving 4D-tomographic image data can comprise extracting for the different time points individual 3D-tomographic image data from the 4D-Tomographic image data. This extraction can be essential for dissecting the 4D data into its constituent 3D components, enabling the analysis and/or segmentation of anatomical structures or pathological processes at specific moments in time. The extraction of 3D-tomographic image data can comprise data decomposition. Data decomposition involves taking the 4D-tomographic image data, which represents a dynamic dataset, and decomposing it into individual frames corresponding to different time points. These frames are essentially 3D-tomographic image data slices, each capturing the examined subject's state at a specific moment during the imaging process. Extracting the 3D-tomographic image data can be based temporal information. The 4D data contains temporal information, such as the time intervals between each 3D image acquisition. This information is critical for associating each 3D image with the precise time point it represents. Extracting the 3D-tomographic image data can comprise synchronization. The extraction process can ensure synchronization of the individual 3D images with the exact moments in the imaging sequence. This synchronization is vital for accurate temporal assessment, especially in applications like cardiac imaging, where the heartbeat's phase must be precisely determined.

The extracted 3D-tomographic image data can be in the same format as the original 4D data, typically in DICOM or another medical imaging format. Alternatively, it may be saved in a format optimized for temporal analysis and display.

The segmentation algorithm is especially a computational method designed to separate or delineate specific structures or regions of interest within medical images, such as 3D-tomographic image data. The goal is to identify and outline the boundaries of these structures, making them distinct from the surrounding tissues or areas. The segmentation algorithms are preferably configured to operate by analyzing pixel or voxel values in the 3D-tomographic image data to distinguish one type of tissue or organ from another.

For example, the segmentation algorithm can be based on thresholding, where an intensity threshold is set, and all pixels or voxels above or below this threshold are assigned to the segmented organ. More complex segmentation algorithms can include region growing, where a seed point is chosen, and the algorithm iteratively adds neighboring pixels or voxels with similar intensities to the segmented region. Another approach for a segmentation algorithm, known as active contour models or snakes, employs deformable shapes that evolve to fit the organ's boundary by minimizing an energy function based on image features.

In particular the segmentation algorithm can comprise machine learning-based approaches, such as deep learning convolutional neural networks (CNNs). For example, the segmentation algorithm is trained on labeled medical images and can efficiently perform segmentation tasks by learning patterns and features within the data.

The input data for the segmentation algorithm is the 3D-tomographic image data containing voxel values that represent anatomical structures. Especially, for each time point 3D-tomographic image data related to this time point are input data for the segmentation algorithm. The output can be a binary mask or labeled map that delineates the organ of interest within the original image. Pixels or voxels belonging to the segmented organ are marked as **"1,"** while those outside are marked as **"0.".** Output can also be a 3D-Model, a 3D-shape or a 3D-Contour for the segmented organ.

The implementation of the segmentation algorithm can be done through dedicated medical imaging software, automation scripts, or custom software developed in programming languages like Python or MATLAB. Preferably, the segmentation algorithm is configured to segmentate the organs without manual intervention.

The scoring function is applied to the segmented organs in 3D-tomographic image data. In other words, input to the scoring function is the segmented organ and/or output from the segmentation algorithm. Input to the scoring function is preferably the 3D-tomographic image data, including or additional to the segmented organ. The scoring function is configured to determine a scoring value for the segmented organ to which it is applied, where the scoring value comprises and/or corresponds to a metric quantifying the extent to which a vicinity of voxels at the surface of the segmented organ in the 3D-tomographic image data contains an image artifact. Segmented Organs could include the lungs, heart, liver, kidneys, or other internal organs. The segmented organ can include the segmented tumor.

The scoring function is an algorithm or method applied to the segmented organs. It assesses the degree to which image artifacts are present in the vicinity of the voxels on the organ's surface. The scoring function can be based on various techniques and algorithms to detect and assess image artifacts. The scoring function is preferably configured as a machine learned and/or trained function. Preferably, the scoring function is applied to each segmented organ separately. Alternatively, the scoring function is applied to a plurality or all segmented organs in a 3D-tomographic image data. The metric is preferably a mathematical formula or procedure used to quantify the extent of image artifacts. This metric may be based on various parameters, such as artifact intensity, their spatial distribution, or other features.

The scoring value generated by the scoring function is preferably a numerical indicator. A higher value may indicate the presence of more image artifacts near the organ's surface, while a lower value would suggest fewer image artifacts. Alternatively, the scoring value is a string value or mixed character value.

Image artifacts are unwanted disturbances or irregularities in medical images. They can be caused by factors such as patient motion during acquisition, image noise, metallic implants, or other issues. These image artifacts can compromise the accuracy of diagnosis and analysis.

In the step comparing the scoring values with a threshold value, the scoring values obtained from the previously described scoring function are compared to a predefined threshold value. The threshold value is a predetermined limit or criterion that serves as a reference point for evaluating the quality of the segmented organs in the 3D-tomographic images. If a scoring value surpasses this threshold, it indicates that the image contains a significant level of image artifacts, and further action may be necessary. The threshold value can be an organ specific value, e.g., lung and heart have different threshold values.

For example, the threshold value is set at 0.7, and a specific organ's scoring value is calculated to be 0.8. This indicates that the image of that particular organ has a high degree of image artifacts, exceeding the predefined threshold.

Once the comparison is performed, and it is determined that one or more of the scoring values have exceeded the threshold value, a user notification is generated. This notification serves to alert the user or operator of the 3D-tomographic imaging system that there are significant image artifacts present in the segmented organs, requiring attention or further investigation. For example, a radiologist or technician operating a medical imaging device receives an immediate notification on their workstation when the scoring value for a patient's lung segmentation surpasses the threshold value of 0.7. This notification prompts the user to review the images for potential image artifacts or retake the scan for a more accurate diagnosis.

In summary, this process ensures that when the quality of segmented organs and/or the quality of segmented subsections of an organ of in 3D-tomographic images falls below an acceptable threshold due to image artifacts, the system generates notifications to alert the relevant users, enabling them to take appropriate actions for a more accurate and reliable assessment.

In particular, the 4D-tomographic image data comprises a number N of 3D-tomographic image data, wherein the segmentation algorithm is applied to the N 3D-tomographic image data, wherein in the step of applying the scoring function at least N scoring values are determined. Especially, for each timepoint and/or for each 3D-tomographic image data at least one scoring value is determined. Particularly, this process involves the analysis of 4D-tomographic image data by applying a segmentation algorithm to a sequence of 3D images and subsequently using a scoring function to evaluate the quality of each segmentation. By generating one or more scoring values for each image, it becomes possible to track changes in image quality over time or across different 3D images in the dataset, which can be crucial for accurate diagnostic or research purposes.

Preferably, the 4D-tomographic image data refers to a collection of sequential 3D-tomographic images captured over time. For instance, in medical imaging, a 4D dataset could represent a series of CT scans taken at various phases of a patient's breathing cycle. Each 3D-tomogrpahic image data within this dataset represents the same anatomical region but at different time points. For example, in cardiac imaging, a 4D-tomographic image data could include 20 3D-tomographic image data of the heart, each acquired at a different phase of the cardiac cycle. N represents the total number of individual 3D-tomographic image data, especially of 3D-tomographic images, within the 4D dataset. It signifies the extent of data available for analysis and segmentation.

Especially, the segmentation algorithm is applied to N 3D-tomographic image data, wherein N is an integer number. The number N is preferably between 5 and 100 and especially between 10 and 25. The segmentation algorithm is used to identify and delineate the regions of interest (e.g., organs or structures) within each of the N 3D-tomographic images. This process results in segmentations specific to each individual image within the dataset. For example in the context of lung imaging, the segmentation algorithm is applied to each of the 100 3D CT scans within the 4D dataset to outline the lung over a breathing cycle. Following the segmentation, the scoring function is applied to each of the N segmented 3D-tomographic images. The scoring function quantifies the extent of image artifacts near the surface of the segmented organs in each image. For example after segmenting the lung in each of the 100 3D CT scans of a patient, the scoring function is applied to assess the presence and extent of image artifacts at the liver's surface in each image. For each time point or for each of the N 3D-tomographic images, one or more scoring values are determined. The number of scoring values corresponds to the number of segmented images and represents the quality of each segmentation. For example, in the imaging of the lung, a scoring value is determined for each of the 100 3D images taken at different time points, allowing assessment of the image quality and potential motion artifacts at each phase.

Particularly, the segmentation algorithm is configured to segment M organs in the 3D-tomographic image data to which it is applied, wherein M is an integer number and/or M equal or larger than **2,** preferably equal or larger than **5.** Preferably, the segmentation algorithm is configured to segment all organs in the 3D-tomographic image data. Optionally, the segmentation algorithm is configured to segment the M most contrasty organs. In the step of applying the scoring function for each timepoint and/or each 3D-tomographic image data at least M scoring values are determined. In other words, for each of the M segmented organs per time point and/or per 3D-tomographic image data, a scoring value is determined. In particular, for a segmentation algorithm applied to N 3D-tomographic image data and configured to segment M organs per 3D-tomogpraphic image data or per timepoint N*M scoring values are determined.

Preferably, the segmentation algorithm is configured to generate a 3D-contour and/or 3D-Volume of the segmented organ, wherein the step applying a segmentation algorithm comprises providing the generated 3D-contour and/or 3D-Volume to the step applying the scoring function. A 3D-contour, short for three-dimensional contour, refers especially to a three-dimensional representation of the outer boundary or surface of a segmented object. It is akin to tracing the edges of an object within the 3D space, outlining its shape in a way that can be easily visualized and measured. A 3D-volume, also known as a three-dimensional volume, represents especially the interior of a segmented object, effectively filling the outlined space with volumetric data. In the context of medical imaging, it can provide a detailed representation of the object's internal structure and can be used for various quantitative analyses. Once the segmentation algorithm has produced the 3D-contour and/or 3D-volume, this data is then forwarded or inputted into the scoring function. The scoring function processes this information.

According to a preferred embodiment of the invention, the 4D-tomographic image data comprise a plurality of segmentable organs, wherein the segmentation algorithm is configured to segmentate a subsection of the plurality of segmentable organs of the 4D-tomographic image data, wherein the subsection of segmentable organs comprises the organs with highest contrast and/or most error prone to motion artefacts. Plurality of segmentable organs means especially, that the 4D-tomographic image data encompasses multiple organs or anatomical structures that can be isolated and analyzed individually. In medical imaging, various organs within the field of view can be segmented or separated for detailed examination. A subsection in this context represents a portion or selection from the larger set of segmentable organs in the 4D-tomographic image data. High contrast, in medical imaging, refers to the clear and distinct differentiation between two or more tissues or structures in an image. Organs or structures that have high contrast are easily distinguishable from their surroundings. For example, in a CT scan, bones often have high contrast because they appear bright white, making them stand out against soft tissues. In tomographic imaging, motion artifacts occur when there is unwanted movement during image acquisition. Certain organs or structures are more susceptible to these artifacts due to their location and inherent mobility. For example, structures near areas of the body with significant motion, such as the diaphragm, can also be affected by motion artifacts.

The scoring function comprises a local Hough transform and/or the scoring function is configured as local Hough transform. The local Hough transform is configured to detect lines and/or direction of lines, wherein the scoring factor determined in the step of applying the scoring function is based on several detected lines and/or directions of detected lines. A local Hough transform is a variation of the traditional Hough transform, which is a mathematical technique used in computer vision and image processing to detect geometric shapes, patterns, or specific features within an image. The local Hough transform, as the name suggests, focuses on a localized or specific region within an image and is often used to find patterns or structures that are not necessarily global in nature. The Hough transform process begins with an input image **(e.g.,** the 3D-tomographic image data) that contains the region of interest or the area where you want to identify specific patterns or structures. This region could contain lines, curves, circles, or other geometrical shapes of interest. Unlike the standard Hough transform, which considers the entire image, the local Hough transform focuses on a specific region or area of interest within the image. This localized region is chosen to limit the computational effort and to target the detection of patterns in a specific area. The localized region can be the segmented organ, the volume or contour of the segmented organ. Especially, the localized region is the surface and/or an area around the surface of the segmented organ. Within the chosen local region, the local Hough transform accumulates information about the presence of certain patterns or structures. For example, the transform accumulates evidence of potential lines in the region. The local Hough transform can create an accumulation space, which is a data structure that stores information about patterns and their parameters. In the case of line detection, this might represent different angles and distances for lines. Each pixel in the local region votes for patterns it believes it has found. After the accumulation step, the local Hough transform can identify peaks in the accumulation space. Peaks correspond to patterns or structures that have received the most votes from pixels in the local region. The peaks in the accumulation space correspond to the parameters of the detected patterns. In the case of line detection, these parameters might include the angle and distance of the detected lines. The output of the local Hough transform is the detection of patterns within the chosen region. This output typically includes information about the parameters of the detected patterns, such as their position, orientation, and size. These parameters can then be used for further analysis or processing, such as object recognition, feature extraction, or other computer vision tasks.

In particular, the scoring function comprises a local Hough transform, wherein the local Hough transform is configured to detect lines and/or direction of lines, wherein in the step of applying the scoring function the Hough transform is applied to the segmented organ and to a vicinity of the segmented organ, wherein the determined scoring factor is based on a comparison of the results of applying the Hough transform to the segmented organ and to the vicinity. The local Hough transform is preferably designed to find lines in the image and potentially determine their directions. When applying the scoring function, they use the Hough transform not just on the segmented organ itself but also in the area around the segmented organ, which is referred to as its **"vi**cinity." This suggests they are interested in not just what's inside the organ but also what's around it. The final score is preferably calculated based on a comparison between what the Hough transform finds within the segmented organ and what it finds in the vicinity. In summary, this embodiment describes a method that involves using a specific technique, the local Hough transform, to analyze images of a segmented organ and the area around it. The scoring function calculates a score based on the results of this analysis.

Particularly, the scoring function is configured to evaluate a local image contrast along the organ boundary of the segmented organ, wherein by applying the scoring function to segmented organ a scoring value corresponding to a stack transition artifact is determined when the local image contrast has a sharp transition and/or a scoring value corresponding to motion artifacts is determined when the local image contrast has a blurred transition. The scoring function is preferably set up to analyze the local image contrast specifically along the boundary of the segmented organ. Image contrast refers to the difference in intensity between adjacent pixels in an image. When the scoring function detects a sharp transition in local image contrast along the organ boundary, it assigns a scoring value that corresponds to a "stack transition artifact." A stack transition artifact could be something like a sudden change in the layers or slices of an image stack, which may indicate a problem in the imaging process or the structure being imaged. On the other hand, when the scoring function identifies a blurred transition in local image contrast along the organ boundary, it assigns a scoring value that corresponds to "motion artifacts." Motion artifacts in medical imaging can result from patient movement during scanning, leading to blurriness or distortion in the image.

Optionally, the step comparing the scoring values comprises, generating the user notification when at least one scoring value exceeds the threshold. The user notification can be an optical, acoustic and/or haptic signal. Preferably, the user notification comprises the time point related to the 3D-tomographic image data with the image artifact, the related the 3D-tomographic image data and/or an overlay image, wherein the overlay image comprises the related 3D-tomographic image an indication of the image artifact and/or the location of the image artifact. In other words, the user notification preferably indicates to the user the 3D-tomographic image data related to the scoring value. This provides a very efficient way to show the user the problematic 3D-tomographic image data, without scanning through all 3D-tomographic image data of the 4D-tomographic image data.

The threshold value can be predefined or user definable, especially the threshold value is an organ specific value. If at least one of the scoring values is higher than the threshold, it triggers a user notification.

The notification may include the original 3D-tomographic image data, which allows the user to see the unaltered image. It might also include an overlay image, which is a modified version of the image that highlights or indicates the presence and location of the image artifact. The overlay image is designed to help the user identify the image artifact or its location. It can include visual cues such as markers or labels that make the artifact more visible.

Preferably, the segmentation algorithm and/or the scoring function is configured as a machine learned algorithm and/or machine learned function. The segmentation algorithm and/or the scoring function can be based on machine learning techniques, where the algorithms or functions are trained on data to improve their performance. The segmentation algorithm can be a deep learning model, like a convolutional neural network (CNN), that has been trained on a large dataset of medical images to accurately identify and outline specific organs within the images. The scoring function can be a machine-learned function that evaluates image quality and/or segmentation based on learned patterns and features. Machine-learned algorithm refers to an algorithm that has been trained on data to perform a specific task.

In particular, the step proving the user notification comprising, providing and/or showing the user notification to/on a scanner console of a scanner used to acquire the 4D-tomographic image data. This step involves making sure that the user notification, which could include important information about the imaging process or the quality of the data, is delivered directly to the scanner console where the operator or user is interacting with the imaging equipment.

Furthermore, the invention is related to a computer program product comprising a computer-readable medium storing a computer program code that, when executed by a computer processor, configures said computer processor to perform the method as claimed in any of the previous claims. The computer program product is especially configured to perform when executed by a computer processor the steps:
- Receiving 4D-tomographic image data, wherein said 4D-tomographic image data comprises a plurality of 3D-tomographic image data of an examination object, wherein the plurality of 3D-tomographic image data corresponds to a plurality of time points,
- Applying a segmentation algorithm to the plurality of 3D-tomographic image data, wherein the segmentation algorithm is configured to segmentate at least one organ in the 3D-tomographic image data to which the algorithm is applied,
- Applying a scoring function to the segmented organs of the 3D-tomographic image data, wherein the scoring function is configured to determine a scoring value for the segmented organ to which it is applied, wherein the scoring value comprises and/or corresponds to a metric quantifying an extent to which a vicinity of voxels at a surface of the segmented organ in the 3D-tomographic image data contains an image artifact,
- Comparing the scoring values with a threshold value,
- Providing a user notification, when at least one scoring value exceeds the threshold value.

The invention is furthermore related to a scanner device. The scanner device is configured to perform the method according to the invention. Furthermore, the scanner device is configured to execute the computer program and/or computer program code of the computer program product according to the invention. The scanner device comprises an interface module and a processor module. The interface module can be a hardware or software module. The processor module can also be a hardware module or a software module, e.g., in cloud computing.

The interface module is configured to receive 4D-tomographic image data, wherein said 4D-tomographic image data comprises a plurality of 3D-tomographic image data of an examination object, wherein the plurality of 3D-tomographic image data corresponds to a plurality of time points.

The processor module is configured to apply a segmentation algorithm to the plurality of 3D-tomographic image data, wherein the segmentation algorithm is configured to segmentate at least one organ in the 3D-tomographic image data to which the algorithm is applied.

Furthermore, the processor module is configured to apply a scoring function to the segmented organs of the 3D-tomographic image data, wherein the scoring function is configured to determine a scoring value for the segmented organ to which it is applied, wherein the scoring value comprises and/or corresponds to a metric quantifying an extent to which a vicinity of voxels at a surface of the segmented organ in the 3D-tomographic image data contains an image artifact,
The processor module is configured to compare the scoring values with a threshold value. The processor module and/or the interface module is configured to provide a user notification, when at least one scoring value exceeds the threshold value.

Further embodiments and/or advantages are described and/or shown in the figures. The figures show:
- Figure 1: a flowchart of a method for assessing the exploitability of 4D-tomographic image data;
- Figure 2: a scanner device for executing the method;
- Figure 3: a system as part of the scanner device.

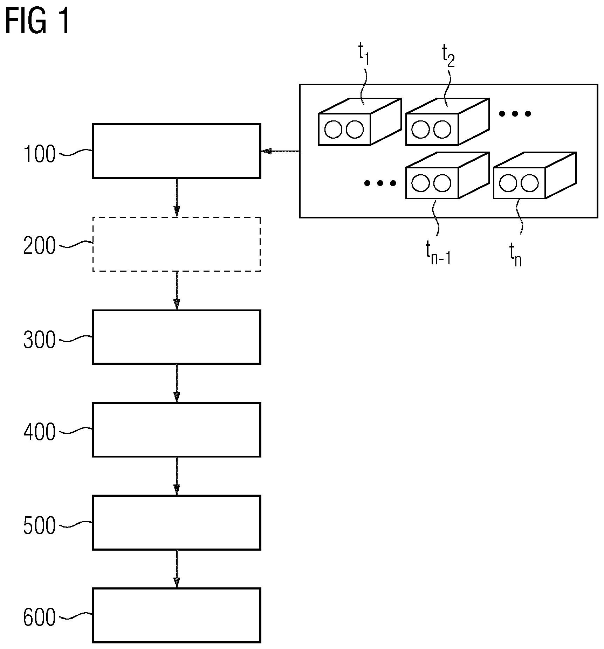

Figure 1 illustrates a flowchart depicting an example of a method for assessing the exploitability of 4D-tomographic image data, with reference sign 4D. This method can be executed by various means, including a scanner device 1(e.g., a computer tomograph), a computer, a processor, or a cloud-based system. Its purpose is to determine the quality of acquired tomographic image data, especially 4D-tomographic data, especially to determine their usability for radiotherapy planning and delivery. Ideally, this method is performed while the examination object, typically a patient, is still within the scanner device 1nor shortly after the acquisition of 4D-tomographic image data 4D. This allows users to assess the suitability of the recently acquired data and decide whether to repeat the scan or data acquisition.

In Step 100, the 4D-tomographic image data 4D are received. These data can be acquired using various medical imaging devices, such as computer tomographs or magnetic resonance tomographs, and are related to an examination object, typically a human patient. The 4D-tomographic image data 4D provide a three-dimensional representation of the region of interest within the examination object over time, with each set of 3D-tomographic image data corresponding to a specific time point. In this example, the 4D-tomographic image data 4D are collected over at least one complete cycle of the patient.

The method may optionally include Step 200, which involves extracting the 3D-tomographic image data from the 4D-tomographic image data. For each time point, the related 3D-tomographic image data extracted are extracted and subsequently processed. These 3D data, particularly the extracted ones, are then passed to Step 300.

In Step 300, a segmentation algorithm is applied to each of the 3D-tomographic image data extracted from the 4D-tomographic image data. This segmentation algorithm, which may be a machine learning algorithm, is designed to identify and outline the contours of one or more organs within the 3D-tomographic image data extracted 3D. Ideally, the segmentation algorithm targets a specific area, such as a user-defined region of interest or an area related to the examination. It is preferable to segment organs with high contrast in the 3D-tomographic image data. After applying the segmentation algorithm to the multiple 3D data sets, segmented organ data, often represented as 3D models, are obtained.

In Step 400, a scoring function, which could be a machine-learned scoring function, is applied to the segmented organs from Step 300. This scoring function is applied to each of the 3D-tomographic image data sets, particularly to the segmented organs within these data sets. The scoring function assesses the extent to which the vicinity of voxels contains image artifacts and provides a scoring value that quantifies this extent. The scoring function may include a Hough transformation. The scoring function determines and/or considers factors like the presence, number, and orientation of lines on the organ's surface or its vicinity.

The scoring values obtained from Step 400 are then used in Step 500, where they are compared to at least one threshold value. This comparison may involve summing, weighting, or averaging the scoring values per organ or per 3D-tomographic image data set. If a scoring value exceeds the threshold value, it indicates a high probability of image artifacts related to the segmented organ and, by extension, the associated 3D-tomographic image data.

In Step 600, a user notification is generated if at least one scoring value exceeds the threshold value. This notification should include the time point corresponding to the 3D-tomographic image data containing the segmented organ with the high scoring value. Optionally, the notification can display the 3D-tomographic image data with an overlay indicating the area and segmented organ with the image artifact. This information helps the user, typically the scanner operator or a medical professional, decide whether to repeat the acquisition of the 4D-tomographic image data for that specific time point or if the identified image artifact is not critical for further use of the data.

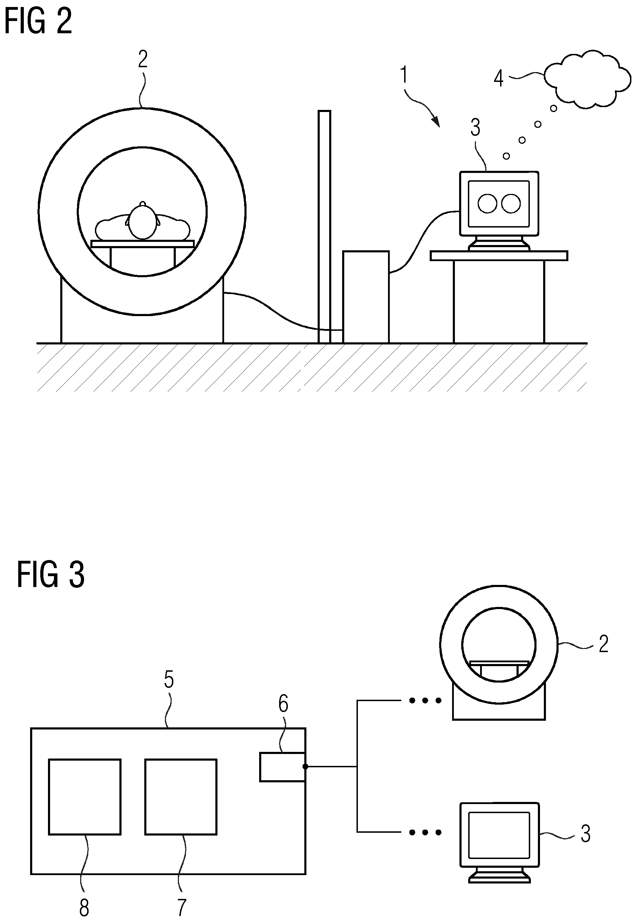

Figure 2 provides an example of a scanner device 1, consisting of a tomography unit 2 and a scanner console 3 connected for data exchange. The scanner console 3 may also be linked to the cloud 4 for data storage or processing outsourcing. The patient or examination subject is scanned by the tomography unit 2, where 4D-tomographic image data 4D are acquired and transmitted to the scanner console 3 and/or the cloud 4.

Figure 3 depicts a system 5, which is a part of the scanner device 1, especially of the scanner unit 2, the scanner console 3 and/or the clod 4. The system 5 executes the method for evaluating the exploitability of 4D-tomographic image data. This system 5 comprises an interface unit 6, a processor unit 7, and a storage unit 8. The interface unit 6 is connected to the tomography unit 2 and the scanner console 3, facilitating data exchange. The processor unit 7 is responsible for executing the steps 200, 300, 400 and/or 400 of the method. The storage unit 8 stores the received 4D-tomographic image data 4D, the extracted 3D data 3D, segmented organs, scoring values, and user notifications.

When a scoring value exceeds the threshold, the processor unit 7 uses the interface unit 6 to send a user notification to the scanner console 3. The user, typically a radiologist, medical physicist, or radiotherapist, can then make an informed decision regarding whether to repeat the acquisition of 4D-tomographic image data 4D.

## Claims

1. A computer implemented method for evaluating the exploitability of 4D-tomographic image data (4D), comprising the steps of:
- Receiving (100) 4D-tomographic image data (4D), wherein said 4D-tomographic image data (4D) comprises a plurality of 3D-tomographic image data (3D) of an examination object, wherein the plurality of 3D-tomographic image data (3D) corresponds to a plurality of time points,
- Applying (300) a segmentation algorithm to the plurality of 3D-tomographic image data (3D), wherein the segmentation algorithm is configured to segment at least one organ in the 3D-tomographic image data (3D) to which the algorithm is applied,
- Applying (400) a scoring function to the segmented organs of the 3D-tomographic image data (3D), wherein the scoring function is configured to determine a scoring value for the segmented organ to which it is applied, wherein the scoring value comprises and/or corresponds to a metric quantifying an extent to which a vicinity of voxels at a surface of the segmented organ in the 3D-tomographic image data (3D) contains an image artifact, wherein the scoring function comprises a local Hough transform, wherein the local Hough transform is configured to detect lines and/or direction of lines, wherein the scoring factor determined in the step of applying the scoring function is based on a number of detected lines and/or directions of detected lines,
- Comparing (500) the scoring values with a threshold value,
- Providing (600) a user notification, when at least one scoring value exceeds the threshold value.

2. A method according to claim 1, wherein the 4D-tomographic image data (4D) comprises a number N of 3D-tomographic image data (3D), wherein the segmentation algorithm is applied to the N 3D-tomographic image data (3D), wherein in the step (400) of applying the scoring function at least N scoring values are determined.

3. A method according to claim 1 or 2, wherein the segmentation algorithm is configured to segment M organs in the 3D-tomographic image data (3D) to which it is applied, wherein in the step (400) of applying the scoring function for each timepoint and/or each 3D-tomographic image data (3D) at least M scoring values are determined.

4. A method according one of the previous claims, wherein the segmentation algorithm is configured to generate a 3D-contour and/or 3D-Volume of the segmented organ, wherein the step applying a segmentation algorithm comprises providing the generated 3D-contour and/or 3D-Volume to the step (400) applying the scoring function.

5. A method according to one of the previous claims, wherein the 4D-tomographic image data (4D) comprise a plurality of segmentable organs, wherein the segmentation algorithm is configured to segment a subsection of the plurality of segmentable organs of the 4D-tomographic image data (4D), wherein the subsection of segmentable organs comprises the organs with highest contrast and/or most error prone to motion artefacts.

6. A method according to one of the previous claims, wherein the scoring function comprises a local Hough transform, wherein the local Hough transform is configured to detect lines and/or direction of lines, wherein in the step of applying the scoring function the Hough transform is applied to the segmented organ and to a vicinity of the segmented organ, wherein the determined scoring factor is based on a comparison of the results of applying the Hough transform to the segmented organ and to the vicinity.

7. A method according to one of the previous claims, wherein the scoring function is configured to evaluate local image contrasts along the organ boundary of the segmented organ, wherein by applying the scoring function to the segmented organ a scoring value corresponding to a stack transition artifact is determined when the local image contrast has a sharp transition and/or a scoring value corresponding to a motion artifacts is determined when the local image contrast has a blurred transition.

8. A method according to one of the previous claims, wherein the step (600) comparing the scoring values comprises generating the user notification when at least one scoring value exceeds the threshold, wherein the user notification comprises the time point related to the 3D-tomographic image data with the image artifact, the related the 3D-tomographic image data (3D) and/or an overlay image, wherein the overlay image comprises the related 3D-tomographic image (3D) an indication of the image artifact and/or the location of the image artifact.

9. A method according to one of the previous claims, wherein the segmentation algorithm and/or the scoring function is configured as a machine learned algorithm and/or machine learned function.

10. A method according to one of the previous claims, wherein the step (600) proving the user notification comprises providing and/or showing the user notification to/on a scanner console of a scanner used to acquire the 4D-tomographic image data.

11. A computer program product comprising a computer-readable medium storing a computer program code that, when executed by a computer processor, configures said computer processor to perform the method as claimed in any of the previous claims.

12. Scanner device comprising a processor unit (7) configured to perform the method according to one of the claims 1 - 10.

## Patentansprüche

1. Computerimplementiertes Verfahren zur Bewertung der Verwertbarkeit 4D-tomographischer Bilddaten (4D), umfassend die folgenden Schritte:
- Empfangen (100) 4D-tomographischer Bilddaten (4D), wobei die 4D-tomographischen Bilddaten (4D) eine Vielzahl von 3D-tomographischen Bilddaten (3D) eines Untersuchungsobjektes umfassen, wobei die Vielzahl von 3D-tomographischen Bilddaten (3D) einer Vielzahl von Zeitpunkten entspricht,
- Anwenden (300) eines Segmentierungsalgorithmus auf die Vielzahl von 3D-tomographischen Bilddaten (3D), wobei der Segmentierungsalgorithmus dazu ausgelegt ist, mindestens ein Organ in den 3D-tomographischen Bilddaten (3D), auf die der Algorithmus angewendet wird, zu segmentieren,
- Anwenden (400) einer Scoring-Funktion auf die segmentierten Organe der 3D-tomographischen Bilddaten (3D), wobei die Scoring-Funktion dazu ausgelegt ist, einen Scoring-Wert für das segmentierte Organ, auf das sie angewendet wird, zu bestimmen, wobei der Scoring-Wert ein metrisches Quantifizieren eines Ausmaßes, bis zu dem eine Umgebung von Voxeln an einer Oberfläche des segmentierten Organs in den 3D-tomographischen Bilddaten (3D) ein Bildartefakt enthält, umfasst und/oder diesem entspricht, wobei die Scoring-Funktion eine lokale Hough-Transformation umfasst, wobei die lokale Hough-Transformation dazu ausgelegt ist, Linien und/oder eine Richtung von Linien zu erfassen, wobei der Scoring-Faktor, der im Schritt des Anwendens der Scoring-Funktion bestimmt wurde, auf einer Anzahl von erfassten Linien und/oder Richtungen erfasster Linien basiert,
- Vergleichen (500) der Scoring-Werte mit einem Schwellenwert,
- Bereitstellen (600) einer Benutzerbenachrichtigung, wenn mindestens ein Scoring-Wert den Schwellenwert übersteigt.

2. Verfahren nach Anspruch 1, wobei die 4D-tomographischen Bilddaten (4D) eine Anzahl N von 3D-tomographischen Bilddaten (3D) umfassen, wobei der Segmentierungsalgorithmus auf die N 3D-tomographischen Bilddaten (3D) angewendet wird, wobei im Schritt (400) des Anwendens der Scoring-Funktion mindestens N Scoring-Werte bestimmt werden.

3. Verfahren nach Anspruch 1 oder 2, wobei der Segmentierungsalgorithmus dazu ausgelegt ist, M Organe in den 3D-tomographischen Bilddaten (3D), auf die er angewendet wird, zu segmentieren, wobei im Schritt (400) des Anwendens der Scoring-Funktion für jeden Zeitpunkt und/oder alle 3D-tomographischen Bilddaten (3D) mindestens M Scoring-Werte bestimmt werden.

4. Verfahren nach einem der vorhergehenden Ansprüche, wobei der Segmentierungsalgorithmus dazu ausgelegt ist, eine 3D-Kontour und/oder ein 3D-Volumen des segmentierten Organs zu erzeugen, wobei der Schritt des Anwendens eines Segmentierungsalgorithmus das Bereitstellen der erzeugten 3D-Kontour und/oder des erzeugten 3D-Volumens für den Schritt (400) des Anwendens der Scoring-Funktion umfasst.

5. Verfahren nach einem der vorhergehenden Ansprüche, wobei die 4D-tomographischen Bilddaten (4D) eine Vielzahl von segmentierbaren Organen umfasst, wobei der Segmentierungsalgorithmus dazu ausgelegt ist, einen Unterabschnitt der Vielzahl von segmentierbaren Organen der 4D-tomographischen Bilddaten (4D) zu segmentieren, wobei der Unterabschnitt der segmentierbaren Organe die Organe mit dem höchsten Kontrast und/oder die für Bewegungsartefakte am fehleranfälligsten Organe umfasst.

6. Verfahren nach einem der vorhergehenden Ansprüche, wobei die Scoring-Funktion eine lokale Hough-Transformation umfasst, wobei die lokale Hough-Transformation dazu ausgelegt ist, Linien und/oder eine Richtung von Linien zu erfassen, wobei im Schritt des Anwendens der Scoring-Funktion die Hough-Transformation auf das segmentierte Organ und auf eine Umgebung des segmentierten Organs angewendet wird, wobei der bestimmte Scoring-Faktor auf einem Vergleich der Ergebnisse des Anwendens der Hough-Transformation auf das segmentierte Organ und die Umgebung basiert.

7. Verfahren nach einem der vorhergehenden Ansprüche, wobei die Scoring-Funktion dazu ausgelegt ist, lokale Bildkontraste entlang der Organgrenze des segmentierten Organs zu evaluieren, wobei durch Anwenden der Scoring-Funktion auf das segmentierte Organ ein Scoring-Wert, der einem Stapelübergangsartefakt entspricht, bestimmt wird, wenn der lokale Bildkontrast einen scharfen Übergang hat und/oder ein Scoring-Wert, der einem Bewegungsartefakt entspricht, bestimmt wird, wenn der lokale Bildkontrast einen unscharfen Übergang hat.

8. Verfahren nach einem der vorhergehenden Ansprüche, wobei der Schritt (600) des Vergleichens der Scoring-Werte das Erzeugen der Benutzerbenachrichtigung umfasst, wenn mindestens ein Scoring-Wert den Schwellenwert übersteigt, wobei die Benutzerbenachrichtigung den Zeitpunkt bezogen auf die 3D-tomographischen Bilddaten mit dem Bildartefakt, die zugehörigen 3D-tomographischen Bilddaten (3D) und/oder ein Überlagerungsbild umfasst, wobei das Überlagerungsbild das zugehörige 3D-tomographische Bild (3D), einen Hinweis auf das Bildartefakt und/oder die Position des Bildartefaktes umfasst.

9. Verfahren nach einem der vorhergehenden Ansprüche, wobei der Segmentierungsalgorithmus und/oder die Scoring-Funktion als maschinell erlernter Algorithmus und/oder maschinell erlernte Funktion ausgelegt ist/sind.

10. Verfahren nach einem der vorhergehenden Ansprüche, wobei der Schritt (600) des Bereitstellens der Benutzerbenachrichtigung das Bereitstellen und/oder Anzeigen der Benutzerbenachrichtigung auf einer Scannerkonsole eines zum Erfassen der 4D-tomographischen Bilddaten verwendeten Scanners umfasst.

11. Computerprogrammprodukt, umfassend ein computerlesbares Medium, das einen Computerprogrammcode speichert, der, wenn durch einen Computerprozessor ausgeführt, den Computerprozessor zur Durchführung des Verfahrens nach einem der vorherigen Ansprüche auslegt.

12. Scannervorrichtung, umfassend eine Prozessoreinheit (7), die dazu ausgelegt ist, das Verfahren nach einem der Ansprüche 1-10 durchzuführen.

## Revendications

1. Un procédé mis en œuvre par ordinateur pour évaluer l'exploitabilité d'une donnée (4D) d'image de tomodensitométrie en 4D, comprenant les stades de :
- recevoir (100) une donnée (4D) d'image de tomodensitométrie en 4D, dans lequel ladite donnée (4D) d'image de tomodensitométrie en 4D comprend une pluralité de données (3D) d'image de tomodensitométrie en 3D d'un objet à examiner, dans lequel la pluralité de données (3D) d'image de tomodensitométrie en 3D correspond à une pluralité de points dans le temps,
- appliquer (300) un algorithme de segmentation à la pluralité de données (3D) d'image de tomodensitométrie en 3D, dans lequel l'algorithme de segmentation est configuré pour segmenter au moins un organe dans les données (3D) d'image de tomodensitométrie en 3D, auxquelles l'algorithme est appliqué,
- appliquer (400) une fonction d'évaluation aux organes segmentés des données (3D) d'image de tomodensitométrie en 3D, dans lequel la fonction d'évaluation est configurée pour déterminer une valeur d'évaluation pour le segment segmenté, auquel elle est appliquée, dans lequel la valeur d'évaluation comprend et/ou correspond à une métrique quantifiant une mesure dans laquelle une proximité de voxels à une surface de l'organe segmenté dans les données (3D) d'image de tomodensitométrie en 3D contient un artefact d'image, dans lequel la fonction d'évaluation comprend une transformée locale de Hough, dans lequel la transformée locale de Hough est configurée pour détecter des lignes et/ou des directions de ligne, dans lequel le facteur d'évaluation déterminé dans le stade d'application de la fonction d'évaluation repose sur un nombre de lignes détectées et/ou de directions de lignes détectées,
- comparer (500) les valeurs d'évaluation à une valeur de seuil,
- donner (600) une notification à un utilisateur, lorsqu'au moins une valeur d'évaluation dépasse la valeur de seuil.

2. Un procédé suivant la revendication 1, dans lequel la donnée (4D) d'image de tomodensitométrie en 4D comprend un nombre N de données (3D) d'image de tomodensitométrie en 3D, dans lequel l'algorithme de segmentation est appliqué aux N données (3D) d'image de tomodensitométrie en 3D, dans lequel dans le stade (400) d'application de la fonction d'évaluation au moins N valeurs d'évaluation sont déterminées.

3. Un procédé suivant la revendication 1 ou 2, dans lequel l'algorithme de segmentation est configuré pour segmenter M organes dans les données (3D) d'image de tomodensitométrie en 3D auquel il est appliqué, dans lequel, dans le stade (400) d'application de la fonction d'évaluation pour chaque point dans le temps et/ou chaque donnée (3D) d'image de tomodensitométrie en 3D, au moins M valeurs d'évaluation sont déterminées.

4. Un procédé suivant l'une des revendications précédentes, dans lequel l'algorithme de segmentation est configuré pour créer un contour en 3D et/ou un volume en 3D de l'organe segmenté, dans lequel le stade d'application d'un algorithme de segmentation comprend donner le contour en 3D et/ou le volume en 3D créé au stade (400) d'application de la fonction d'évaluation.

5. Un procédé suivant l'une des revendications précédentes, dans lequel la donnée (4D) d'image de tomodensitométrie en 4D comprend une pluralité d'organes segmentables, dans lequel l'algorithme de segmentation est configuré pour segmenter une sous-partie de la pluralité des organes segmentables de la donnée (4D) d'image de tomodensitométrie en 4D, dans lequel la sous-partie d'organes segmentables comprend les organes ayant le contraste le plus grand et/ou ayant le plus tendance à l'erreur dû à des artéfacts de mouvement.

6. Un procédé suivant l'une des revendications précédentes, dans lequel la fonction d'évaluation comprend une transformée locale de Hough, dans lequel la transformée locale de Hough est configurée pour détecter des lignes et/ou des directions de lignes, dans lequel dans le stade d'application de la fonction d'évaluation la transformée de Hough est appliquée à l'organe segmenté et à une proximité de l'organe segmenté, dans lequel le facteur d'évaluation déterminé repose sur une comparaison des résultats d'application de la transformée de Hough à l'organe segmenté et à la proximité.

7. Un procédé suivant l'une des revendications précédentes, dans lequel la fonction d'évaluation est configurée pour évaluer des contrastes locaux d'image le long de la limite de l'organe segmenté, dans lequel en appliquant la fonction d'évaluation à l'organe segmenté, une valeur d'évaluation correspondant à un artefact de transition d'empilement est déterminée, lorsque le contraste local d'image a une transition nette et/ou une valeur d'évaluation correspondant à des artéfacts de mouvement est déterminée, lorsque le contraste local d'image a une transition floue.

8. Un procédé suivant l'une des revendications précédentes, dans lequel le stade (600) comparant les valeurs d'évaluation comprend créer la notification à un utilisateur, lorsqu'au moins une valeur d'évaluation dépasse le seuil, dans lequel la notification à un utilisateur comprend le point dans le temps se rapportant aux données d'image de tomodensitométrie en 3D avec l'artefact d'image, les données (3D) d'image de tomodensitométrie en 3D en relation et/ou une image de recouvrement, dans lequel l'image de recouvrement comprend l'image (3D) de tomodensitométrie en 3D en relation, une indication de l'artefact d'image et/ou l'emplacement de l'artefact d'image.

9. Un procédé suivant l'une des revendications précédentes, dans lequel l'algorithme de segmentation et/ou la fonction d'évaluation est configurée sous la forme d'un algorithme d'apprentissage par machine et/ou d'une fonction d'apprentissage par machine.

10. Un procédé suivant l'une des revendications précédentes, dans lequel le stade (600) donnant la notification à un utilisateur comprend donner et/ou montrer la notification à un utilisateur à/sur une console d'un scanner utilisé pour acquérir la donnée d'image de tomodensitométrie en 4D.

11. Un produit de programme d'ordinateur comprenant un support pouvant être déchiffré par ordinateur mémorisant un code de programme d'ordinateur, qui, lorsqu'il est exécuté par un processeur informatique, configure ledit processeur informatique pour effectuer le procédé tel que revendiqué dans l'une quelconque des revendications précédentes.

12. Dispositif de scanner comprenant une unité (7) de processeur configurée pour effectuer le procédé suivant l'une des revendications 1 à 10.
